# NEW EUROPEAN PATENT SPECIFICATION

(11) **EP 1 171 633 B2**
(45) Date of publication and mention of the opposition decision: **18.03.2015**
(45) Mention of the grant of the patent: 03.01.2007
(21) Application number: 00917213.1
(22) Date of filing: 11.04.2000
(51) Int. Cl.: C12Q 1/68

(54) **METHODS OF MARKING MATERIALS**
METHODE ZUR MARKIERUNG VON MATERIALIEN
PROCEDES DE MARQUAGE DE MATERIAUX

(30) Priority: 13.04.1999 GB 9908437
(43) Date of publication of application: 16.01.2002
(73) Proprietor: TraceTag International Limited, Cardiff CF2 7HE (GB)
(72) Inventor: MINTON, John Edward, Lisvane, Cardiff CF14 0TF (GB); SLEAT, Robert, Rhiwbina, Cardiff CF14 6JP (GB)
(74) Representative: Arends, William Gerrit
(86) International application number: PCT/GB2000/001367
(87) International publication number: WO 2000/061799

(56) References cited:
- WO-A-89/07272
- WO-A-92/02638
- WO-A-94/04918
- WO-A-95/02702
- WO-A-97/46714
- WO-A-99/42613
- REISCHL U ET AL: "QUANTITATIVE PCR A SURVEY OF THE PRESENT TECHNOLOGY" MOLECULAR BIOTECHNOLOGY,US,TOTOWA, NJ, vol. 3, 1995, pages 55-71, XP000600241 ISSN: 1073-6085

## Description

This invention relates to methods for marking materials, and the detection of such marking.

There is a widespread requirement to be able to trace the path taken by a given material as it moves from one location to another. The movement may be of natural materials (for example, the flow of water in sub-surface aquifers) or materials which have been processed or manufactured by man (for example, any article constructed by man in a manufacturing process or natural resources such as grains and minerals). In these situations there may be reasons why it is necessary to develop specific procedures to trace these movements. It may be that direct observation is not possible, as when following the path of a stream underground. It may be that it is necessary to monitor the movement of goods without the direct knowledge of the transporters or, for legal reasons, to prove that the appearance of a material at a particular point in the biosphere was due to the same material originating from a known starting point.

For example, articles of manufacture may be stolen in transit or resold at a much lower price than that set by the supplier by an unscrupulous distributor, for example at car boot sales. A key problem in bringing a conviction is identification of the particular articles sold, to establish that the goods have been stolen or resold from a particular distributor. Problems also occur with liquids such as petroleum which are routinely washed out of carriers into the sea. It is almost impossible to identify which carrier has discharged the oil and as such prosecutions and convictions for polluting the seas are rarely brought. Further problems are associated with the movement of natural materials, for example the movement of grain. It is particularly difficult to distinguish one batch of such natural materials from another. In the case of grain, problems occur in the European Community with the grain being moved across several different borders to collect a number of EU subsidies for the same batch of grain. A method of marking the grain which may be readily detected is necessary to prevent such fraud.

Various methods are known in the art for marking and detecting materials, many of which make use of microtrace nucleic acid labels, particularly labels made of DNA. Nucleic acids can provide a limitless amount of information, because of the variable sequence of bases (adenine, cytosine, guanine and thymine [uracil in the case of RNA which replaces thymine]) contained within the molecule. Probability terms can be calculated for the frequency of a given sequence of bases and, so long as sufficient bases are used, i.e., a sufficiently large DNA molecule is employed as the tag, then for all practical purposes a unique microtrace can be defined. By using combinations of universal sequences (accepted as industrial standards) and by varying levels of specific sequences, it is possible to identify the type of generic product, the product's origin (company specific sequences), the lot or batch, and even provide an identifier for a unit of commerce.

Previous International Patent Application No. PCT/GB91/00719 (published as WO91/17265) disclosed a method of monitoring the movement of a hydrocarbon by the addition of hydrocarbon compatible DNA as a microtrace additive. International Application No. PCT/GB93/O1822 (published as WO94/04918) discloses a method of marking a liquid and subsequently detecting that the liquid has been marked which comprises adding an additive to the liquid. The additive comprises two or more types of particles not visible to the naked eye, each having different signal means or particles having two or more different signal means. The signal means aid in the detection of the microbeads. One of the signal means comprises nucleic acid, while the other signal is not a nucleic acid tag. In each of the above methods, the unique microtrace comprising DNA molecules is added to the material, the resulting material is sampled after movement thereof, and the presence of the microtrace additive in the sample is detected, analysed and decoded.

International Application No. PCT/GB94/01506 (published as WO95/02702) describes a method of marking a solid article or item by adding to a liquid an additive comprising microbeads not visible to the naked eye comprising two or more signal means to aid their detection and code means to aid identification. The additive may comprise either two or more microbeads each having different signal means, at least one microbeads having a code means, or it may comprise a microbead having two or more different signal means and at least one code means. The code means and one of the signal means comprises a nucleic acid and another of said signal means comprises a non-nucleic acid signal means. The liquid containing the additive is added to the solid and allowed to dry in order to mark the solid. Subsequently, microbeads having signal means are detected on the solid, the solid is sampled and the code means decoded. The method can be adapted for use in monitoring an interaction between any material, article or item and a person or animal, by making use of a device adapted to produce an aerosol containing a liquid comprising an additive containing a plurality of microbeads.

International application No. PCT/GB93/01822 (published as WO94/04918) discloses a method of marking a liquid and subsequently detecting that the liquid has been marked. The method comprises: adding to the liquid an additive comprising a plurality of particles in an amount no greater than 1 part weight of particles per 10⁶ parts weight liquid, the particles comprising signal means to aid their detection and not being visible in the liquid to the naked eye; sampling a position of the liquid containing the additive, and detecting the presence of particles in the liquid, with the proviso that the signal means does not consist solely of a nucleic acid tag.

In each of the above prior art methods, the microtrace nucleic acid or DNA disclosed is a synthetic nucleic acid or DNA having a variable region flanked by regions having pre-determined sequences. The sequence of the variable region gives each microtrace nucleic acid or DNA molecule a unique, characteristic identity, while the pre-determined sequences are common to all tags. The pre-determined sequences are recognised by primers for amplification by the polymerase chain reaction (PCR) and are used for subsequent sequencing. Thus, in order to determine the identity of the variable region in the microtrace nucleic acid or DNA, the variable region is amplified using primers complementary to the flanking regions having pre-determined sequences. The variable region is then sequenced using the same primers, to determine the sequence of the variable region and hence the identity of the tag. Alternatively, sequencing can be initiated from a sequencing primer region incorporated into the tag between a flanking region and the variable region. As each sequencing primer region can have a sequence unique to a particular tag, this allows the use of two or more different nucleic acid or DNA tags to mark the material.

Although the procedures described above are effective, the prior art methods essentially provide only a "YES/NO" answer. In other words, they are able to determine whether a particular sample contains a nucleic acid tag, and what the identity of the tag is. As far as we are aware, however, no one has previously appreciated that it may be desirable to determine the quantity of tag present in a marked material. The methods described in the prior art have been concerned solely with the marking, detection and identification of tags, without providing any indication how much of a nucleic acid tag is present in the sample.

We have now found that it may be advantageous to determine how much of a marker or tag is present in a marked material. We have recognised that the quantity of a tag in a material can give valuable clues as to the past history and movements of the material.

For example, by measuring the actual quantity of a tag in a material and comparing this to the amount initially used to mark the material, it is possible to tell whether a customer (whether deliberately or inadvertently) has diluted the material. The quantity of tag in the material can also indicate whether contamination has taken place, and the appropriate action can then be taken. Determining the quantity of a particular marker is also useful when there are multiple sources of an environmental contamination. Here, different suspected sources of contamination, for example, material at different factories or plants, may be marked by different tags. A sample is taken from the polluted effluent stream, and the presence of each of the different tags may be detected to determine if the pollution is caused by a particular factory. Importantly, by measuring the relative concentrations of each tag in the effluent stream, information is provided on the relative contribution of each source to the environmental pollution. Appropriate fines may then be imposed by the authorities on the polluting factories or plants with the knowledge of the contribution of each factory or plant to the pollution.

There is therefore value in a method of marking a material and subsequent detection which provides an indication of the quantity of marker present in the material, and this has not been previously appreciated in the field.

It will also be appreciated that there are inherent problems with the prior art techniques. In particular, several steps are involved in the detection of marker in the sample. First of all, PCR reactions have to be carried out to amplify the microtrace DNA tag. In order to determine if the reactions are successful, and to resolve the reaction products from primer-dimers, unincorporated primers and nucleotides, the reaction products may then be subjected to polyacrylamide gel electrophoresis, for example. The reaction products are then excised from the gel and the amplified DNA purified from the gel fragments. The purified DNA is then subjected to cycle sequencing to determine the sequence of the variable regions. Finally, the sequence needs to be interpreted and compared with a known database to determine the identity of the tag used. The many steps involved in the detection phase of the prior art methods make the procedure lengthy, time-consuming and laborious and therefore expensive to undertake.

Furthermore, for adequate separation of the PCR reaction products from primer-dimers, unincorporated primers and deoxynucleotides, the length of the amplified DNA should be relatively long. In general, experience shows that the amplified DNA should be greater than 200 base pairs in length for good separation to be achieved using a normal resolution gel. The need for a relatively long amplification product for good separation needs to be balanced by the expense and difficulty involved in designing and synthesising long (>100 nucleotides long) oligonucleotides for use as the tag. Therefore, although it is more efficient and less expensive to synthesise smaller tags, it is correspondingly more difficult to distinguish these small tags from amplification artefacts following PCR.

There is therefore also an unfulfilled need for a method of marking a material and subsequent detection which involves fewer steps and which can make use of relatively short oligonucleotides as tags.

There is disclosed herein a method of detecting whether a material has been marked by a marker comprising a nucleic acid tag, the method comprising the steps of: (a) sampling a portion of the material; and (b) detecting the presence of the nucleic acid tag in the sample, said method characterised in that the quantity of nucleic acid tag present in the sample is determined to provide an indication of the quantity of marker present in the material.

The method may also comprise the prior step of: adding or applying a marker comprising a nucleic acid tag to the material.

The nucleic acid tag may be amplified by means of a nucleic acid amplification reaction. The quantity of nucleic acid tag present in a sample may be determined by measuring the amount of amplification needed for the amount of amplified nucleic acid to reach a pre-determined level. The amount of amplified nucleic acid in a nucleic acid amplification reaction may be determined in a number of ways. The actual means by which the amount of amplified nucleic acid is determined is not important, although we will describe here a number of preferred ways by which the amount may be measured: It will be appreciated that the more nucleic acid tag is initially present in a sample, the less amplification is required to reach a particular pre-detemnined level of amplified nucleic acid. It is therefore possible to calibrate the detection system using known initial quantities of nucleic acid tags in a series of test reactions. The kinetics of accumulation of amplified nucleic acid in a reaction can then be measured, and compared to that of the test reactions. This allows one to determine how much nucleic acid tag is present in a test sample, and hence how much nucleic acid is present in the marked material. Various methods of nucleic acid amplification are known, for example, polymerase chain reaction, ligase chain reaction, TMA, and NASBA, etc, and these methods can be employed for the amplification of nucleic acid tags in the methods described herein. However, the nucleic acid amplification reaction preferably used is the polymerase chain reaction (PCR).

We envisage that the methods described here are suitably used with different embodiments of nucleic acid tag, each of which contains at least one identifying sequence for the tag. When we refer in this application to an "identifying sequence", we mean a arrangement of nucleotides having a particular sequence, which sequence enables the identity of the nucleic acid tag in which it is present to be determined. A pool of nucleic acid tags may thus be generated, each of which has one or more different identifying sequence(s). The remainder of each tag in the pool (in other words, regions not including the identifying sequence or sequences) can have sequences common to all nucleic acid tags in the pool. After a material is marked by a tag from the pool, a sample of material can be taken. Detecting the presence of a particular identifying region in the sample therefore provides a direct indication of the identity of the nucleic acid tag used to mark the material.

In the detection methods, the nucleic acid tag is contacted with two oligonucleotide primers in a nucleic acid amplification reaction. A first nucleic acid tag (referred to here as a Type I tag) contains only one identifying sequence, and only one of the oligonucleotide primers has a sequence which corresponds to this identifying sequence. In another nucleic acid tag (referred to as a Type II tag), two identifying sequences are present, and both oligonucleotide primers have sequences corresponding to respective identifying sequences. In other words, it may be said that a Type II tag is amplified using primers to its identifying sequences. In another alternative tag, which we will refer to as a Type III tag, only one identifying sequence is present which is flanked by primer regions, and neither of the oligonucleotide primers has a sequence corresponding to the identifying sequence.

The nucleic acid tag can therefore be said to be contacted with a first oligonucleotide primer capable of priming amplification of the nucleic acid tag and having a sequence corresponding to a first sequence contained in the nucleic acid tag. In a first nucleic acid tag (Type I), the first sequence is the same sequence as the identifying sequence, and the first oligonucleotide primer therefore has a sequence corresponding to the identifying sequence. The other oligonucleotide primer in the amplification reaction may correspond to any other sequence in the nucleic acid tag. All that is essential is that one of the oligonucleotide primers is capable of priming from the identifying sequence, so that the presence of nucleic acid amplification indicates that the identifying sequence is present, and hence provides an indication of the identity of the nucleic acid tag.

In a Type II tag, the nucleic acid tag also comprises a second identifying sequence, in which case the nucleic acid tag is further contacted in the amplification reaction with a second oligonucleotide primer having a sequence corresponding to the second identifying sequence. The first oligonucleotide may have a sequence consisting of the first identifying sequence and the second oligonucleotide a sequence complementary to the second identifying sequence. Alternatively, the first oligonucleotide has a sequence complementary to the first identifying sequence and the second oligonucleotide a sequence consisting of the second identifying sequence. The two identifying sequences may be adjacent to each other, or they may flank intervening sequences. For a Type II tag, amplification takes place in the presence of two oligonucleotide primers, and both primers must be able to bind during the amplification reaction in order for successful amplification to take place. The two identifying sequences in a Type II tag may have identical sequences, or they may have different sequences. The latter arrangement allows for a greater number of permutations of individually distinct nucleic acid tags to be produced.

It will be apparent that, where an amplification reaction is set up with at least one oligonucleotide primer corresponding to an identifying sequence or sequences of a Type I or Type II nucleic acid tag, the specificity of the amplification reaction essentially resides in the particular oligonucleotide primer or primers used. If amplification takes place, this means that the oligonucleotide primer or primers has recognised a complementary identifying sequence in the nucleic acid tag. Conversely, absence of amplification indicates that the particular identifying sequence is not present in the tag present in the sample. This allows for the identity of an unknown nucleic acid tag to be easily determined by setting up a series of nucleic acid amplification media with different primers, and detecting which of the amplification media results in successful amplification. It will be appreciated that the methods using the above nucleic acid tags allow for a "one-step" procedure to be performed, in that detection, identification and quantitation of nucleic acid tag takes place in one reaction. The presence of amplification indicates that nucleic acid tag is present in the sample, and gives an indication of the identity of the nucleic acid tag used to mark the material. At the same time, the quantity of amplified nucleic acid can be measured to give an indication of the quantity of nucleic acid tag in the sample and hence the material. There is therefore no need for further purification of amplified tag and subsequent sequencing to determine identity. The nucleic acid tags used can thus be much shorter than those previously used.

There is also disclosed herein a method of marking a material and subsequently detecting that it has been marked, the method comprising the steps of: (a) adding or applying a marker comprising a nucleic acid tag to the material, the nucleic acid tag comprising at least one identifying sequence for the tag; (b) sampling a portion of the material containing such marker and optionally separating the nucleic acid tag from the sample; (c) amplifying at least a portion of the nucleic acid tag by means of a nucleic acid amplification reaction which includes contacting the nucleic acid tag with a first oligonucleotide having a sequence corresponding to the identifying sequence of the nucleic acid tag; and (d) detecting amplification of nucleic acid as an indication of the presence of marker in the sample and hence that the material has been marked.

There is also disclosed herein a method of detecting whether a material has been, marked by a marker comprising a particular nucleic acid tag, the nucleic acid tag comprising at least one identifying sequence for the tag, the method comprising the steps of: (a) sampling a portion of the material and optionally separating any nucleic acid tag present from the sample; (b) amplifying any nucleic acid tag present in the sample by means of a nucleic acid amplification reaction which includes contacting the nucleic acid tag with a first oligonucleotide having a sequence corresponding to the identifying sequence of the nucleic acid tag; and (c) detecting amplification of nucleic acid as an indication of the presence of the particular nucleic acid tag in the sample and hence that the material has been marked by a marker comprising that tag.

The nucleic acid tag may comprise at least one identifying sequence for the tag, and the method additionally comprises the steps of: (c) amplifying at least a portion of the nucleic acid tag by means of a nucleic acid amplification reaction which includes contacting the nucleic acid tag with a first oligonucleotide having a sequence corresponding to the identifying sequence of the nucleic acid tag; and (d) determining the amount of amplification required for the amplified nucleic acid to reach a pre-determined level as an indication of quantity of marker present in the sample.

Conveniently, the method additionally comprises the steps of: (c) amplifying at least a portion of the nucleic acid tag by means of a nucleic acid amplification reaction which includes contacting the nucleic acid tag with a first oligonucleotide having a sequence corresponding to the identifying sequence of the nucleic acid tag; and (d) determining the amount of amplification required for the amplified nucleic acid to reach a pre-determined level as an indication of quantity of marker present in the sample and hence the quantity of marker in the material.

A third nucleic acid tag (Type III) has one identifying sequence which is flanked by first and second regions of the nucleic acid tag containing priming sites for nucleic acid amplification. The Type III tag is therefore amplified in a nucleic acid amplification reaction by contacting it with a first oligonucleotide primer having a sequence which does not correspond to the identifying sequence of the tag. The other oligonucleotide primer in the amplification reaction has a sequence which also does not correspond to the identifying sequence. Thus, the first oligonucleotide has a sequence which corresponds to a sequence contained in either the first or the second region and the second oligonucleotide contains a sequence corresponding to a sequence in the other of the first and second regions of the tag.

For this tag, a pool of nucleic acids can be generated with the first and second regions being common to all tags in the pool, but with the identifying sequence distinct for each member of the pool. Nucleic acid amplification can be conducted using oligonucleotide primers having "generic" sequences, which bind to sequences in the first and second regions of the tag. Detection of the identifying sequence and quantitation of amplified nucleic acid can take place contemporaneously with the amplification, or afterwards.

There is disclosed herein a method of marking a material and subsequently detecting that it has been marked, said method characterised in that it provides an indication of the quantity of marker present in the material, the method comprising the steps of: (a) adding or applying a marker comprising a nucleic acid tag to the material, the nucleic acid tag comprising first and second regions flanking an identifying sequence for the tag; (b) sampling a portion of the material containing such marker and optionally separating the nucleic acid tag from the sample; (c) amplifying at least a portion of the nucleic acid tag by means of a nucleic acid amplification reaction which includes contacting the nucleic acid tag with a first oligonucleotide having a sequence corresponding to said first region of the nucleic acid tag; and (d) determining the amount of amplification required for the amplified nucleic acid to reach a pre-determined level as an indication of quantity of marker present in the sample. The nucleic acid amplification reaction may be a polymerase chain reaction.

There is disclosed herein a method of marking a material and subsequently detecting that it has been marked, the method comprising the steps of: (a) adding or applying a marker comprising a nucleic acid tag to the material, the nucleic acid tag comprising first and second regions flanking an identifying sequence for the tag; (b) sampling a portion of the material containing such marker and optionally separating the nucleic acid tag from the sample; (c) contacting the nucleic acid tag with a first oligonucleotide primer having a sequence corresponding to a sequence contained in said first region of the nucleic acid tag and a oligonucleotide probe bearing a signal means and having a sequence corresponding to said identifying sequence; (d) amplifying at least a portion of the nucleic acid tag by means of a polymerase chain reaction which includes a nucleic acid polymerase having 5' to 3' exonuclease activity in which fragments bearing signal means are released from the oligonucleotide probe during nucleic acid amplification; and (e) detecting released fragments as an indication that amplification has taken place and hence that the material has been marked wherein the quantity of nucleic acid tag present in the sample is determined to provide an indication of the quantity of marker present in the sample. The nucleic acid tag may be contacted with a second oligonucleotide primer capable of priming amplification of the tag and having a sequence corresponding to a sequence contained in the second region. In addition to having signal means, which may be a fluorescent label, the oligonucleotide probe may also bear quenching means to attenuate a signal from signal means. Such attenuation is preferably a result of Förster resonance energy transfer through space. A detectable signal from a released fragment may be at a substantially higher level than a signal detectable from the oligonucleotide probe.

In accordance with the invention, we provide a method of detecting wether a material has been marked with a nucleic acid tag, the method comprising the steps of:
(a) providing a pool of nucleic acid tags, each nucleic acid tag comprising a generic region having a sequence present in all nucleic acid tags within the pool, the generic region being flanked by code regions having identifying sequences for the tag;
(b) selecting a particular nucleic acid tag from within the pool;
(c) adding or applying a marker comprising the selected nucleic acid tag to the material; and
(d) contacting the nucleic acid tag with a labelled oligonucleotide probe capable of binding the generic region of the nucleic acid tag; and
(e) detecting hybridisation of the oligonucleotide probe to the generic region of the nucleic acid tag, thereby indicating that the material was marked with the nucleic acid tag.

There is disclosed herein a method of determining which particular nucleic acid tag from a known pool of different nucleic acid tags has been used to mark a material, each nucleic acid tag in the pool comprising at least one identifying sequence for the tag, the method comprising the steps of: (a) sampling a portion of the marked material and optionally separating the nucleic acid tag from the sample; (b) setting up a plurality of nucleic acid amplification reaction media, each medium containing an oligonucleotide having a sequence corresponding to the identifying sequence of a different known nucleic acid tag in the pool and being capable of amplifying a different nucleic acid tag from the pool; (c) contacting the nucleic acid tag of the sample with each of said reaction media; and (d) detecting which of the reaction media results in the amplification of nucleic acid tag as an indication of the identity of the particular nucleic acid tag used to mark the material wherein the quantity of nucleic acid tag present in the sample is determined to provide an indication of the quantity of marker present in the sample.

There is also disclosed herein a kit for determining which particular nucleic acid tag from a known pool of different nucleic acid tags has been used to mark a material, each nucleic acid tag in the pool comprising at least one identifying sequence for the tag, the kit comprising means for setting up a plurality of nucleic acid amplification reaction media, each medium containing an oligonucleotide having a sequence corresponding to the identifying sequence of a different known nucleic acid tag in the pool and being capable of amplifying a different nucleic acid tag from the pool. The nucleic acid tag comprises a generic region having a sequence present in all nucleic acid tags in the pool, and each nucleic acid amplification medium also preferably comprises an oligonucleotide having a sequence corresponding to a sequence contained in the generic region.

In preferred embodiments of the invention, the marker further comprises an indicator means in addition to the nucleic acid tag, the presence of which is readily detectable in any sampled portion of the material. The presence of the indicator means in a sample indicates that nucleic acid tag is also present in the sample. Thus, a simple test for the presence of indicator means and hence nucleic acid in a sample may be done before detailed analysis of the identity or quantity of the tag is undertaken. The indicator means may comprise a plurality of particles. The particles may be formed of any suitable non-living or non-viable formerly living matter material, and the particles may have any size or shape appropriate for the intended purpose. Generally, particles having an average size not greater than about 5 micrometres are suitable for most purposes. The particles preferably have an average size of from 0.01 to 5 micrometres, more preferably 0.05 to 1 micrometre, with a typical size about 0.25 or 0.5 micrometre. What is important is that they are not visible to the naked eye, but can be detected easily. For this purpose, the particles may be labelled, for example, with fluorescent dye.

Preferably, the indicator means comprises microbeads or microspheres, optionally bearing a readily detectable label. Exemplary microbeads/spheres are commercially available from Dynal (U.K.) of Wirral, Merseyside, UK, under the generic trade names DYNABEADS and DYNASHPERES. The preparation of these beads is disclosed, in, for example, European Patent Publication Nos. 91953, 10986 and 106873 and U.S. Patent Nos. 4186120, 4563510 and 4654267.

The method according to the invention in its aspect as described here can be used for quantitative determination of the amount of nucleic acid tag in a marked material. We describe three preferred methods of quantitating the amount of amplified nucleic acid in a reaction. As mentioned previously, the actual method of detection is not relevant, although there are advantages in using the methods described here.

A preferred embodiment of the invention utilises a 5' nuclease assay for detection and quantitation, which is a modification of the polymerase chain reaction (PCR). The 5' nuclease assay was initially described in Holland et al.; 1991 (Holland, P. M., Abramson, R. D., Watson, R. and Gelfand, D. H, Proc. Natl. Acad Sci. USA 88, 7276-7280) and Holland et al., 1992 (Holland, P. M., Abramson, R. D., Watson, R., Will, S., Saiki, R. K. and Gelfand, D. H, Clinical Chemistry, 38, 462-463). This assay is also the subject of US Patent Nos. 5,210,015 and 5,487,972, and of subsequent modifications. A commercial kit for conducting the 5' nuclease assay, known as TAQMAN, is available from PE Biosystems.

In a 5' nuclease assay as applied here, the nucleic acid tag is contacted with a labelled oligonucleotide probe, in addition to a first oligonucleotide primer having a sequence corresponding to a first sequence in the tag, in the presence of a DNA polymerase having a 5' to 3' exonuclease activity. The labelled oligonucleotide probe is capable of binding to a sequence in the nucleic acid tag during amplification. For the first nucleic acid tag having one identifying sequence (Type I tag), the labelled probe binds to a sequence 5' to 3' downstream of the first oligonucleotide primer. For the second nucleic acid tag (Type II tag), i.e., where the nucleic acid tag has two identifying sequences and is further contacted with a second oligonucleotide primer corresponding to the second identifying region, the sequence which is bound by the probe is flanked by the first and second identifying sequences. Thus, the labelled probe when bound to the nucleic acid tag during amplification lies 5' to 3' downstream of one of the oligonucleotide primers.

For the third tag (Type III tag), the sequence bound by the probe consists of the identifying sequence for the nucleic acid tag. Thus, for this tag, the locations of the first and second regions (containing the priming sites) are such that the first oligonucleotide primer bound to the nucleic acid tag lies 5' to 3' upstream of the labelled probe bound to its corresponding identifying sequence. For all the tags, the first oligonucleotide primer and the probe may lie adjacent to each other. Alternatively, they may be separated by the presence of intervening sequence in the nucleic acid tag. The probe is labelled with a signal means, preferably a fluorescent tag.

During the synthesis phase of the PCR, the first oligonucleotide primer is extended by the polymerase activity of the DNA polymerase. The labelled oligonucleotide probe lying downstream will however be degraded by the 5' to 3' exonuclease activity of the polymerase, and fragments bearing signal means will be released. The signal means on the released fragments may therefore be detected as an indication of the progress of the amplification reaction. In particular, the presence of such a signal indicates that amplified nucleic acid is present. Thus, if a signal is detected this indicates that the nucleic acid is present in the sample and hence that the material has been marked. Furthermore, the amount of signal will be proportional to the amount of amplified nucleic acid produced during the course of the reaction, so that measuring the level of signal will give an indication of the amount of amplified nucleic acid.

Any polymerase having a 5' to 3' nuclease activity may be used in the 5' nuclease assay. Mammalian or bacterial DNA polymerases, for example, DNA polymerase I from *E. coli,* are therefore suitable for use in the method. For polymerase chain reaction, the polymerase should be stable to heat. Thermostable polymerases are well known in the art, for example, DNA polymerases from *Thermus aquaticus, Pyrococcus furiosis, Thermococcus litoralis, Thermus flavus, Thermus thermophilus,* etc.

In addition to being labelled by a signal means, the oligonucleotide probe may optionally also be labelled with a quencher means, which operates to attenuate a signal detectable from the fluorescent tag in an intact probe. It has been found that adequate attenuation occurs when the signal means is at the 5' end of the oligonucleotide and the quencher means is at the 3' end. It is thought that attenuation occurs means of Förster resonance energy transfer through space (FRET, described by Förster, V. Th., (1948), Annals Physics (Leipzig), 255-75). Thus, the proximity of the quenching means to the signal means on the oligonucleotide probe causes a signal detectable from the signal means to be attenuated, when the second oligonucleotide is intact. Such attenuation does not occur when the labelled fragments are released by the exonuclease activity of the polymerase, when the quencher means is separated from the signal means. In this case, the detectable signal from the intact oligonucleotide probe is substantially lower than the detectable signal from the released fragments, and the signal from the released fragments may more readily be detected.

An alternative method of measuring the presence or amount of nucleic acid is by measuring the incorporation of oligonucleotide primers into amplicons. For this purpose, the first oligonucleotide primer, and/or second oligonucleotide primer (if present), is conveniently labelled by a signal means. As amplification progresses, the primer(s) are incorporated into amplified nucleic acid amplicons. A signal from a primer which has been so incorporated is detected as a measure of the presence of amplified nucleic acid in the reaction. Furthermore, such a signal may be measured to provide an indication of the quantity of amplified nucleic acid in the reaction. The primer(s) may optionally be labelled also with a quenching means which if present attenuates a detectable signal from the signal means in a free oligonucleotide primer. When the oligonucleotide is incorporated into an amplicon, however, the quenching means is not able to attenuate the signal from the signal means. Thus, the signal detectable from a free oligonucleotide (in solution) is at a substantially lower level than the signal detectable from an incorporated primer. The signal means may be a fluorescent label and the attenuation may take place by means of Förster resonance energy transfer through space.

Other ways of detecting and determining the amount of amplified nucleic acid are also possible. Thus, for example, the amount of amplified nucleic acid during PCR reaction may be quantitated periodically sampling the reaction. The amount of nucleic acid material in the samples which is hybridisable to a nucleic acid probe is then measured. The probe is a oligonucleotide having a sequence complementary to a sequence contained in the nucleic acid tag, this sequence being flanked by the sequences bound by the primers used in the amplification. It will be appreciated that this method works because only amplified nucleic acid is capable of hybridising to such a probe. The probe is preferably labelled so as to enable it to be detected more readily. Such a detection principle is used in the PCR-LIGHT Quantitative PCR System manufactured by Tropix/PE Applied Biosystems. In this system, one oligonucleotide primer is labelled with biotin, and the PCR reaction is interrupted during the exponential phase of amplification. A sample is taken from the reaction tube, and is bound to a streptavidin coated surface. The bound product is then denatured, and the unbound strand removed. An "internal" probe labelled with fluorescein is then contacted with the denatured PCR product, and an anti-fluorescein antibody conjugated with alkaline phosphatase is added together with a chemiluminescent substrate. Light emission measured in a luminometer indicates the presence and quantity of amplification product.

The nucleic acid forming the tag may be deoxyribonucleic acid (DNA) or ribonucleic acid (RNA). The tag may be made of protein conjugated to nucleic acid (protein-nucleic acid, PNA). DNA is preferred because of its greater stability and resistance to nucleases. Although the nucleic acid tag may be double stranded, it preferably consists of a single stranded DNA molecule, more preferably, an oligonucleotide. Both naturally occurring and synthetic nucleic acids are suitable for use as the tag. The term "naturally occurring" refers to DNA or RNA molecules occurring in nature. The term "synthetic" is applied to DNA or RNA synthesised in the laboratory using routine synthesis procedures well known in the relevant art.

Preferably, the tag is a synthetic DNA oligonucleotide. Synthetic DNA may be formed from the five naturally occurring bases: adenine, thymine, guanine, cytosine and uracil, and non-naturally occurring bases, for example, inosine bases, and derivatized nucleotides, such as 7-deazo-2'deoxyguanosine, alkylphosphonate oligodeoxynucleotides, phosphorothioate oligodeoxynucleotides and α-anomeric oligodeoxynucleotides. In certain circumstances, tags incorporating non-naturally occurring bases may have advantages over those containing only naturally occurring bases, for example, in stability, because they are less likely to be degraded by nuclease activity, or by chemically active substances or by environmental conditions, such as heat or ultraviolet radiation. The use of tags incorporating non-naturally occurring bases is limited only by their ability to be effectively detected by the detection means. For tagging methods using the preferred PCR technology, the tag must be capable of forming duplexes with PCR primers and function as a template for the polymerases used in the PCR procedure.

Preferably, the nucleic acid is detected and quantitated at the same time as being amplified. Various nucleic acid amplification technologies can be used in the method described here, for example, Polymerase Chain Reaction (PCR), Ligase Chain Reaction (LCR), NASBA and TMA. In a preferred embodiment, the method utilises PCR, disclosed in U.S. Patent Nos. 4683202 and 4683195 and European Patent Publication Nos. 258017 and 237362.

Typically, the identifying sequence comprises at least 20 nucleotide bases to ensure adequate specificity for any tag so that accidental contamination will not lead to false results. The longer the sequence, the higher the potential information content of the tag, but the more likely that degradation will become a problem. Furthermore, synthesis of longer tags is more expensive and less efficient, as discussed above. Preferably, the nucleic acid tag is between 60 to 125 base pairs or nucleotides in length. More preferably, the nucleic acid tag is less than 90 nucleotides or base pairs in length and most preferably the nucleic acid tag is about 63 nucleotides or base pairs in length. This permits the hybridisation of two primers which are typically about each 20 bases or nucleotides in length, and which, when hybridised to the tag, are separated by a region in the tag of between about 20 to up to 85 bases/nucleotides in length. This region may contain a binding region for a 5' nuclease probe, as described in further detail below. The actual size of the tag is not important, as long as the tag is of adequate size for primers of suitable size to recognise corresponding sequences within the tag. For reasons of economy, the tags may be chosen to be as short as possible.

The label or signal means may be fluorescent substance, especially a fluorescent dye. Suitable fluorescent dyes include (but are not limited to): allophycocyanine, phycocyanine, phycoerythrine, rhodamine, oxazine, coumarin, fluoroscein derivatives, for example, fluorescein isothiocyanate and carboxyfluoroscein diacetate, as well as Texas red, acridine yellow/orange, ethidium bromide, propidium iodide, bis-benzamide (commercially available from Hoechst under the trade name H33258) etc. Preferably, the fluorescent label is FAM (6-carboxy-fluorescein) or TET (6-carboxy-4,7,2'7'-tetrachloro-fluorescein).

A nucleic acid tag chosen from the pool of tags is used to mark a material by applying a marker comprising the chosen tag to the material. If the material to be marked is a liquid, then it is merely necessary to add the oligonucleotide tag directly to the liquid. If the material to be marked is a water-based liquid, then the tag may be added directly to the liquid. If the material is hydrocarbon or oil based, the tag may be suitably modified (e.g., by methylation) before adding to the material. In the case of a solid material being marked, the oligonucleotide tag may first be dissolved in a suitable liquid, which is then applied to the solid and allowed to dry. The oligonucleotide tag may also be applied in the form of an aerosol, which is sprayed onto the solid. In addition to the oligonucleotide tag, the marker may also additionally comprise a binder or a detection means as described previously.

Subsequently, a sample may be taken from the marked material and directly subjected to analysis, with optional pre-processing if necessary. For example, if the material is a liquid, a small volume of the liquid may be taken and added directly to the amplification reactions. Similarly, small solid particles (e.g., scapings) may also be added directly to the amplification reactions. However, it is usually preferred in the case of a solid material for the nucleic acid tag to be extracted from the solid beforehand. This may conveniently be done by washing the solid material to which the marker has been applied with a suitable water-based buffer.

Further details of these methods will now be described with reference to the accompanying drawings, in which:
FIGURE 1 is a plot of detected fluorescent signal over background at each PCR cycle for a series of starting concentrations (per ml) of nucleic acid tag; and
FIGURE 2 is a logarithmic plot derived from the data in Figure 1, showing the threshold cycle (i.e., the cycle at which the detected signal is above the background level) for a series of starting concentrations of nucleic acid tag.

### Experiment I: Type I structure detected and quantitated with 5' nuclease assay

A Type I tag may be used to mark a material, and identified and quantitated by means of the 5' nuclease assay.

A pool of single stranded DNA oligonucleotide tags is synthesised according to conventional oligonucleotide synthesis methods as known in the art. The tags are preferably about 70 nucleotides (nt) in length, and contain only one identifying sequence within each tag. For example, the pool may contain the following tags:

SEQ ID: NO 1

Region A Region B Region C

ACGTAGTAAAGAGGTGCCCGCCCCGCTTTCGCAGAACACCGAAGGAAGACCACACGTGAGCCCAGAAC

SEQ ID: NO 2

Region A Region B Region C

ACGTAGTAAAGAGGTGCCCGCCCCCGCTTTCGCAGAACACCGAAGGAAGAACTGATCGGGCTCAACTGGA

As can be seen above, the identifying sequence (Region C) is a variable region having a particular sequence which is different from tag to tag. The sequence of this region will therefore be unique to the particular tag on which the particular sequence is disposed. Regions A and B, on the other hand, have the same sequences for all tags in the pool, and can be referred to as "generic" sequences. In this case, if the identifying sequence in a tag is 20 nucleotides long, then with 4 bases available, approximately 1.1 x 10¹² unique tags can be synthesised.

A single oligonucleotide tag is then chosen from the pool and used to mark a material by applying a marker comprising the chosen tag, as described above. Subsequently, a portion of the marked material is sampled, and the sample subjected to analysis to determine the identity and/or the quantity of the tag in the sample. If necessary, the oligonucleotide tag may be optionally extracted from the sample and subjected to analysis.

For the purposes of detection and quantitation, a set of corresponding oligonucleotide primer pairs is synthesised for use in a polymerase chain reaction (PCR) using conventional DNA synthesis methods well known in the art. One oligonucleotide primer in each pair of the set has a sequence consisting of a sequence in Region A of the pool of tags. The other oligonucleotide primer in the pair has a sequence complementary to a sequence in the variable identifying region (Region C) of a different oligonucleotide tag in the pool. Thus, the number of primer pairs corresponds to the number of oligonucleotide tags in the pool, and each primer pair in the set is capable of amplifying one (and only one) of the oligonucleotide tags in the pool. It will be appreciated that the design of the primer pairs and the oligonucleotide probe can be varied. Thus, one oligonucleotide primer in a pair may have a sequence complementary to a sequence in Region A, in which case the other primer will have a sequence consisting of a sequence in Region C.

In addition to the primer pairs, a third oligonucleotide is constructed for use as a probe in the 5'nuclease assay, again using conventional DNA synthesis methods. The oligonucleotide probe has a sequence consisting of or complementary to a sequence in Region B of the tags in the pool. As with the choice of PCR primers, due consideration will have to given to the sequence of the labelled probe. In particular, the probe sequence is chosen so that its melting point is preferably above that of both PCR primers, so that during the amplification reaction, the probe is bound to its target before any significant primer extension takes place.

The oligonucleotide probe is labelled with a fluorescent label such as FAM (6-carboxy-fluorescein) or TET (6-carboxy-4,7,2'7'-tetrachloro-fluorescein). The fluorescent label is attached to the probe by conventional means known in the art. In addition, it is possible nowadays to order from commercial sources custom made probes with fluorescent or other labels attached. As well as the fluorescent label, quenching means is also attached to the probe, for example, by means of any conventional coupling process as known in the art. The quenching means is preferably a quencher dye which, when present on the same oligonucleotide probe as the fluorescent label, attenuates or reduces the fluorescence signal observed from the fluorescent label preferably by Förster resonance energy transfer (FRET). Thus, in an intact oligonucleotide probe, the signal from the fluorescent label is reduced.

A series of 5' nuclease assay reactions is then set up. Each reaction tube contains an aliquot portion of the sample containing the unknown oligonucleotide tag, *Taq* DNA polymerase, dNTPs, magnesium chloride, and buffer. In addition, as PCR primers, each reaction tube contains a different primer pair chosen from the set of oligonucleotide primers described above, as well as an amount of the labelled oligonucleotide probe. The number of reaction tubes corresponds to the number of different oligonucleotide tags in the pool. Since each primer pair of the set is only capable of amplifying one and only one of the oligonucleotide tags in the pool of tags, the fact that a successful PCR reaction has occurred in a particular reaction tube indicates that the primer pair in that tube has recognised corresponding sequences in the oligonucleotide tag and enabled amplification of that tag. The identity of the oligonucleotide tag used to mark the material can therefore easily be determined by running the PCR reactions and determining which of the reactions is successful. Of course, if it is suspected that a sample contains a particular tag, then it is necessary only to run one PCR reaction with the appropriate primer pair to see if amplification takes place. Similarly, if the tag is believed to belong to a small number of candidate tags, a reduced number of reactions can be run with the appropriate primer pairs.

The advantage of the 5' nuclease assay is that quantitation of tag can take place at the same time as detection of amplification. A single fluorescence signal is produced, the presence of which indicates that amplification has taken place (and hence provides the identity of the tag), while the strength of the signal is determined to provide an indication of the amount of tag in the sample (as described in more detail below).

When the labelled probe and the primers are hybridised to their respective complementary sequences, the structure of the tag is such that the labelled probe lies 5' to 3' downstream of one of the primers. As the DNA polymerase extends the primer bound to the tag, it encounters the labelled probe bound to the tag. The 5' to 3' nuclease activity of the polymerase degrades the labelled probe, and releases labelled fragments into the reaction medium. Depending on the structure of the tag, however, the primers and the probe, the labelled probe when hybridised to the tag may even lie immediately downstream (i.e., adjacent) of the primer. In this case, the exonuclease activity proceeds to degrade the labelled probe without any primer extension taking place. As mentioned above, in an intact oligonucleotide probe, the signal from the fluorescent label is reduced, but when labelled fragments are released, the signal is no longer attenuated and may be detected by appropriate means, for example, a fluorescence detector. As amplification progresses, the strength of the signal should increase exponentially, as an increasing number of labelled fragments are released into the medium. The strength of the signal may be determined and used to determine the initial concentration of the tag in the sample as described below. The reaction vessels used in the assay have walls which are transparent to the fluorescent signal, and the signal may therefore be detected during the course of the PCR reaction without the reaction needing to be interrupted.

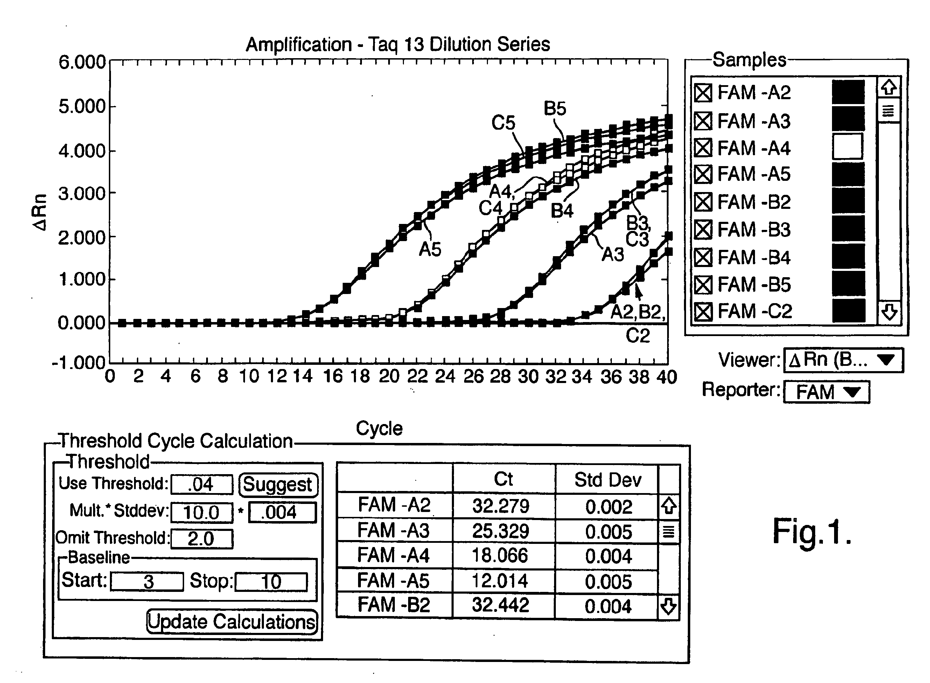

Prior to the amplification reactions being run, the detection system is calibrated using known initial quantities of oligonucleotide tags in a series of test reactions. FIGURE I is a graph showing the level of fluorescent signal detected over the background level (Y axis) at each cycle of the PCR reaction (X axis), for a series of initial concentrations (10⁴, 10⁶, 10⁸, 10¹⁰ per ml) of nucleic acid tag. Oligonucleotides A2, B2 and C2 have concentrations of 10⁴/ml, A3, B3 and C3 have concentrations of 10⁶/ml, A4, B4 and C4 have concentrations of 10⁸/ml while A5, B5 and C5 have concentrations of 10¹⁰/ml. The horizontal bar, shows the background level of fluorescent signal, which is set to be equal to zero. As can be seen, the threshold cycle, in other words the cycle at which the signal becomes detectable over the background level (i.e., the point at which the curve starts to rise), is different for different initial concentrations of tag. The more tag is initially present in the sample, the earlier in the reaction the signal becomes significant. The data used for Figure 1 is tabulated in Table 1 below.

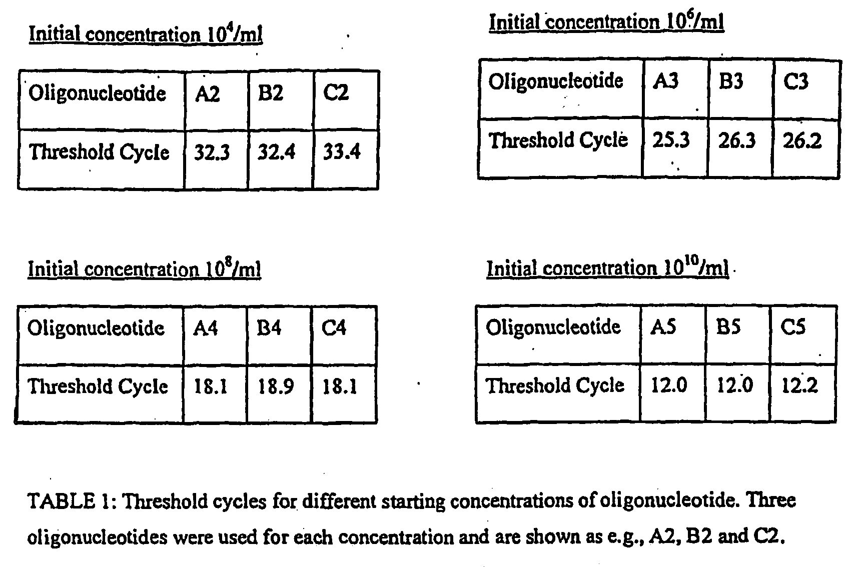

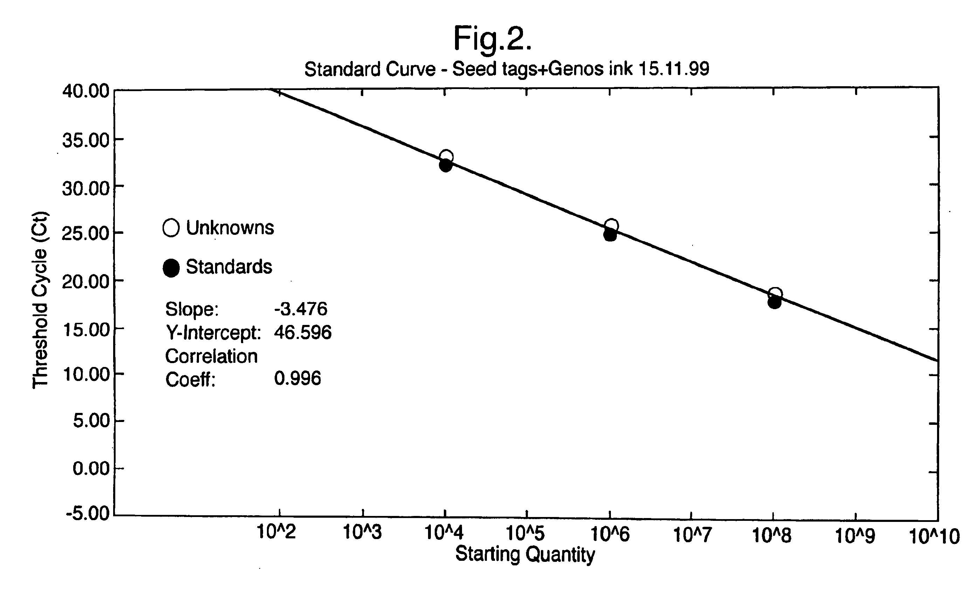

The data gathered during the calibration may be plotted to generate a standard curve. FIGURE 2 is a graph showing the threshold cycle (in other words, the cycle at which the signal is detectable over background) as the Y axis, plotted against the logarithm of the initial starting concentration. It will be seen that the threshold cycle is directly proportional to the log of the initial starting concentration. In an ideal case, the slope of the line should be -3.3.

In order to determine the initial concentration of the tag in the test reaction, the level at which the signal is detectable over background is determined. This threshold cycle is then compared to the standard curve to provide the initial concentration of the tag in the sample. The concentration of the tag in the sample can of course be used to determine the concentration of the tag in the marked material.

### Experiment II: Type II structure detected and quantitated with 5' nuclease assay

A Type II tag may be used to mark a material, and identified and quantitated by means of the 5' nuclease assay.

A pool of single stranded DNA oligonucleotide tags is synthesised according to conventional oligonucleotide synthesis methods as known in the art. The tags are preferably about 70 nucleotides (nt) in length, and contain two identifying sequences within each tag. For example, the pool may contain the following tags:

SEQ ID: NO 3

Region A Region B Region C

ATGACGATGTCGGAATGATCCGACCCGCTTTCGCAGAACACCGAATACCAACTGCGACCAAGGCGT

SEQ ID: NO 4

Region A                    Region B                    Region C

ATTGGAGACAGACCCGAAGCGACCCGCTTTCGCAGAACACCGAAACGGTGGAAATACCAACTGCGA

As shown above, in a Type II tag Regions A and C are variable regions having identifying sequences which are different from tag to tag. Region B, on the other hand, is a "generic" sequence having the same sequence for all tags in the pool. In the above pool, since the identifying sequences are now 40 (2 x 20) nucleotides long, approximately 2.2 x 10¹² different tags are available. Thus, using a Type II tag instead of a Type I tag increases the number of tags available for marking.

The material is marked and sampled in the same way as described above in Example I. The subsequent identification and quantitation of the tag is as also as described above in Example I, except that since there are now two identifying regions, both of each pair of primers in the primer set are designed to prime amplification of a single tag in the pool. In other words, one oligonucleotide primer in a pair of the set has a sequence consisting of a sequence in Region A of a oligonucleotide tag of the pool, while the other primer in the pair has a sequence complementary to a sequence in Region C of the same oligonucleotide tag. Alternatively, one primer has a sequence complementary to a sequence in Region A and the other a sequence consisting of a sequence in Region C. Thus, each pair of primers is capable of amplifying one and only one of the tags in the pool. In addition to the primer pairs, a third labelled oligonucleotide probe is synthesised as described in Experiment I above.

The 5' nuclease assay is conducted in the same way as described in Experiment I.

As with a Type I tag, the presence of a successful amplification provides the identity of the tag, and the level at which the signal is detectable over background is determined and compared with a standard curve to provide the initial concentration of the tag in the sample. Needless to say, the concentration of the tag in the marked material may be calculated easily from the concentration of the tag in the sample.

### Experiment III: Type I or Type II tag detected and quantitated by measuring incorporation of labelled primers into amplicons

A Type I tag or a Type II tag may be used to mark a material, and identified and quantitated by detecting and determining the amount of incorporation of labelled oligonucleotide primer into amplicons (amplification products).

A pool of single stranded DNA oligonucleotide tags is synthesised according to conventional oligonucleotide synthesis methods as known in the art. The tags are preferably about 70 nucleotides (nt) in length, and contain one (Type I) or two (Type II) identifying sequences within each tag. For example, the pool may contain the following Type I tags:

SEQ ID: NO 5

Region A

Region B

ACGTAGTAAAGAGGTGCCCGCCACCCGCTTTCGCAGAACACCGAAGGAAGACCACACGTGAGCCCAGAAC

SEQ ID: NO 6

Region A

Region B

ACGTAGTAAAGAGGTGCCCGCCCCCGCTT TCGCAGAACACC GAAGGAAGAACTGATCGGGCTCAACTGGA

Alternatively, the pool may contain the following Type II tags:

SEQ ID: NO 7

Region A

Region B

ATGACGATGTCGGAATGATCCGACCCGCTTTCGCAGAACACCGAATACCAACTGCGACCAAGGCGT

SEQ ID: NO 8

Region A Region B

ATTGGAGACAGACCCGAAGCGACCCGCTTTCGCAGAACACCGAAACGGTGGAAATACCAACTGCGA

The design and synthesis of the tags, and the marking and sampling of the material, is as described in Example I above. In a Type I tag (SEQ IDs 5 and 6), Region A is the same for all tags in the pool, while Region B is the identifying sequence which is different in each tag in the pool. In the Type II tag, both Regions A and B (SEQ IDs 7 and 8) are identifying sequences. A set of primer pairs is synthesised as described above in Experiment I (in the case of a Type I tag) and Experiment II (in the case of a Type II tag). As before, each pair of primers is capable of amplifying one and only one tag within the pool. At least one of the primers in each pair should be labelled with a signal means (e.g., fluorescent tag) and a quenching means, as described above in relation to the labelled oligonucleotide probe in Experiment I.

A series of PCR amplification reactions is set up as described in Experiment I above, except that the labelled oligonucleotide probe for the 5' nuclease assay is not present in the reaction mix.

The fluorescent tag and quenching means are chosen such that in a free primer in solution, the quenching means attenuates the signal from the fluorescent tag. This attenuation takes place by means of Förster resonance energy transfer through space. When the labelled oligonucleotide primer is extended by the DNA polymerase and incorporated into an amplification product (amplicon) however, the quenching means does not attenuate the signal from the fluorescent tag, and a signal from the fluorescent tag may be detected. As amplification proceeds, the level of detectable signal should increase exponentially. The higher the concentration of tag initially present in the sample, the lower the cycle number at which the signal is detectable over background. By calibrating the detection apparatus in the same manner as described in Experiment I, a standard curve can be produced, which will allow the quantity of tag to be determined. Again, the presence of the signal can be determined at the same time as the level of the signal, to provide the identity of the tag as well as its quantity in the sample.

### Experiment IV: Type I or Type II tag detected and quantitated by directly measuring amplification product

A Type I tag or a Type II tag may be used to mark a material, and identified and quantitated by directly detecting and determining the amount of amplification products produced during the PCR reaction. The amplification products are detected and quantitated using the PCR-LIGHT Quantitative PCR System manufactured by Tropix/PE Applied Biosystems.

A pool of single stranded DNA oligonucleotide tags is synthesised according to conventional oligonucleotide synthesis methods as known in the art. The tags are preferably about 70 nucleotides (nt) in length, and contain one (Type I) or two (Type II) identifying sequences within each tag. For example, the pool may contain the following Type I tags:

SEQ ID: NO 9

Region A Region B Region C

ACGTAGTAAAGAGGTGCCCGCCACCCGCTTTCGCAGAACACCGAAGGAAGACCACACGTGAGCCCAGAAC

SEQ ID: NO 10

Region A Region B Region C

ACGTAGTAAAGAGGTGCCCGCCCCCGCTTTCGCAGAACACCGAAGGAAGAACTGATCGGGCTCAACTGGA

Alternatively, the pool may contain the following Type II tags:

SEQ ID: NO 11

Region A Region B Region C

ATGACGATGTCGGAATGATCCGACCCGCTTTCGCAGAACACCGAATACCAACTGCGACCAAGGCGT

SEQ ID: NO 12

Region A Region B Region C

ATTGGAGACAGACCCGAAGCGACCCGCTTTCGCAGAACACCGAAACGGTGGGAAATACCAACTGCGA

The design and synthesis of the tags is as described in Experiment I above. In a Type I tag (SEQ IDs 9 and 10), "generic" Regions A and B are the same for all tags in the pool, while Region C is the identifying sequence which is different in each tag in the pool. In the Type II tag, both Regions A and C (SEQ IDs 7 and 8) are identifying sequences, while Region B is common between all the tags in the pool. For PCR amplification, a set of primer pairs is synthesised as described above in Experiment I (in the case of a Type I tag) and Experiment II (in the case of a Type II tag). As before, each pair of primers is capable of amplifying one and only one tag within the pool.

To enable the amplification products to be captured, one of the primers in each pair is labelled with biotin.

The marking and sampling of the material is as described in Experiment I above. A series of PCR reactions is set up, each reaction tube containing a different primer pair chosen from the set of oligonucleotide primers, as described in Experiment I. Detection and quantitation of amplification product is effected by periodically taking a sample from each reaction tube during the exponential phase of amplification. The sample is applied to a microplate whose wells are coated with streptavidin. Unincorporated biotin labelled primer as well as any amplification products are captured in the well. The wells are then washed several times with buffer. The amplification products are then denatured to expose single stranded DNA bound to the microplate, and the complementary strands are washed away. A fluorescein labelled probe having a sequence complementary to the generic sequence of Region B is added to the microplate and allowed to hybridise to the bound single stranded DNA. An anti-fluorescein antibody conjugated with alkaline phosphatase is then added together with a chemiluminescent substrate, and light emission detected and measured in a luminometer. The presence of light emission indicates a successful amplification and hence the identity of the nucleic acid tag, while the quantity of light emitted indicates the amount of amplification product. As described above in relation to Experiment I, this can be compared against a standard curve to give the initial concentration of the tag in the sample and hence in the marked material.

### Experiment V: Type III tag detected and quantitated by 5' nuclease assay

A Type III may be used to mark a material, and identified and quantitated by means of the 5' nuclease assay.

A pool of single stranded DNA oligonucleotide tags is synthesised according to conventional oligonucleotide synthesis methods as known in the art. The tags are preferably about 70 nucleotides (nt) in length, and contain one identifying sequence. For example, the pool may contain the following tags:

SEQ ID: NO 13

Region A Region B Region C

ACGTAGTAAAGAGGTGCCCGCCACCCGCTTTCGCAGAACACCGAAGGAAGACCACACGTGAGCCCAGAAC

SEQ ID: NO 14

Region A Region B Region C

ACGTAGTAAAGAGGTGCCCGCCATGGCATCGTCGGAGTTGGAGCTGGAAGACCACACGTGAGCCCAGAAC

In a Type III tag, only one identifying sequence which is different from tag to tag is present (Region B). Regions A and C have sequences which are common to all tags in the pool. A single oligonucleotide tag is chosen from the pool and used to mark a material as described above in Experiment I. Subsequently, a portion of the marked material is sampled and subjected to analysis to determine its identity and/or the quantity of the tag in the sample. For this, a pair of oligonucleotide primers is synthesised one of which has a sequence consisting of a sequence in Region A and the other a sequence complementary to a sequence in Region C. Alternatively, one primer may have a sequence complementary to a sequence in Region A and the other a sequence consisting of a sequence in Region C. In addition, a set of labelled oligonucleotide probes is also synthesised, each probe in the set having a sequence complementary or corresponding to a sequence in Region B of a single tag in the pool. The probes are labelled by using fluorescent tags having different emission maxima. Thus, for example, one probe in the set may be labelled with rhodamine, and another with fluorescein isothiocyanate (FITC). Each probe is also bound to a quenching means, which attenuates the signal from the fluorescent tag in an intact oligonucleotide probe.

To identify and quantitate the tag used to mark the material, a 5' nuclease reaction is set up containing the pair of oligonucleotide primers in addition to a number of differently labelled probes of the set. As the primer pair is complementary to sequences in the "generic" regions of the pool, any tag present in the sample will be amplified whatever its identity. However, only one of the labelled probes will bind to the oligonucleotide tag during the reaction. As described above in Experiment I, a labelled probe hybridised to its complementary sequence in the tag will lie 5' to 3' downstream of a amplification primer hybrised to the tag, and the 5' to 3' exonuclease activity of the *Taq* DNA polymerase will degrade the labelled probe during DNA synthesis to release labelled fragments. As above, the quenching means is not capable of attenuating the signal from a fluorescent tag in a released fragment. Since various fluorescent tags are used to label the different probes present in the 5' nuclease reaction, the fluorescent signal emitted from the 5' nuclease reaction will have one of several wavelengths depending on which particular probe is degraded. In order to detect the emitted signal, therefore, a multichannel detector capable of detecting emitted radiation in a number of different wavelengths is used. Since it is known which fluorophore was used to label which probe, detection of the wavelength (or colour) of the emitted light will indicate which labelled probe was bound to the tag and degraded during the reaction, and hence provide the identity of the oligonucleotide tag within the pool which was used to mark the material. The strength of the signal increases exponentially during a successful reaction, and appropriate calibration of the detection system will allow the initial concentration of the oligonucleotide tag to be determined, as explained above in Experiment I.

It will be appreciated that the advantage of using a 5' nuclease assay with Type III tags is that it is possible theoretically to identify and quantitate the tag by using a single amplification reaction. This technique is limited only by the number of distinct fluorescent tags available to label the different oligonucleotide probes, and by the ability of the multichannel detectors to discriminate between the wavelengths of emitted signal. Current multichannel detectors are capable of detecting up to eight distinct wavelengths, meaning that at present it is possible to have eight differently labelled oligonucleotide probes in the 5' nuclease reaction. Several such reactions therefore need to be carried out depending on the size of the pool of tags. However, as the technology progresses, we expect that it will be possible in the future to have a "one-tube" detection and quantitation process.

### Experiment VI: Detailed Example Illustrating Type II tag in 5' Nuclease Assay

Two stages are involved in the design of oligonucleotide tags, random sequence generation and primer/probe design. In the first stage, a large number of random DNA sequences of 100 bases in length is first generated using PC/Gene software purchased from Oxford Molecular Limited, United Kingdom. Any software capable of generating random DNA sequences may be used. The sequences are chosen to have nominal equal composition for all four bases, and the generated sequences exported into appropriate primer/probe design software for the second stage of primer/probe design.

The software used to design the primers and probe is "Primer Express" from PE Applied Biosystems. "Primer Express" is available as part of the software for operating the PE-ABI 7700 analytical hardware used to undertake 5' nuclease assays, which hardware is also produced and sold by PE Applied Biosystems. The detailed operation of the software is described in the bundled manuals accompanying the software. The "Primer Express" software applies the normal rules for PCR primer design (e.g., the need to avoid hairpins, the need for the primers to have matching Tₘ, etc) to the design of the primers. In addition, the software avoids significant complementarity between the probe and either of the PCR primers and ensures that the probe is complementary to the sequence of interest and binds to a region between the two primers. The software also ensures that the probe has a Tₘ at least 10 degrees Celsius higher than either amplification primer, and does not have a G at the 5' end of the probe.

Once a random oligonucleotide sequence has been imported into the "Primer Express" software, the software is instructed to create a new probe and primer design. The software is then instructed using the "Find Primers/Probe Now" function to determine the first 200 primer and probe combinations conforming to the default set of parameters supplied with the software. The default settings are as follow: minimum Tₘ for both primers is set at 50 degrees C, maximum Tₘ is set at 60 degrees C, and optimal Tₘ is set at 58 degrees C. The maximal Tₘ difference between the primers is 2 degrees. The GC content of the primers is set to have a minimum of 20%, a maximum of 80%, with no 3' GC clamp for either primer. The minimum length of the primer is set to be 9, the maximum 40 and the optimal length is 20. The minimum Tₘ of the amplicon is set to be 0 degrees, the maximum Tₘ is 85 degrees, while the minimum and maximum lengths of the amplicon are 50 and 150 respectively. The probe Tₘ is set to be at least 10 degrees higher than the Tₘ of the primers, and the sequence of the probe is set to not begin with a G. the maximum base repeat of the primers is set at 3 residues, while the primer secondary structure requirements are set to be: maximum consecutive base pair 3, maximum total base pair 8. The primers and probe combination with the lowest penalty score in respect of the oligonucleotide are then identified, and the probe sequence is then used as a basis for designing primers for the other random oligonucleotide sequences as described below.

The "common probe sequence" identified above is introduced into the next random oligonucleotide imported into the "Primer Express" software. For this purpose, the editing functions of the "Primer Express" software are utilised, as described in more detail in the operating manual for the software. Experience has shown that the best place to insert the probe sequence is around base position 35. The inserted probe sequence can then be fixed using the software, and the "Find Primers/Probe Now" routine undertaken again to locate the optimal primer pairs on the resulting sequence (i.e., the random sequence with the probe inserted). It will be appreciated that since the software is instructed to fix the probe sequence, this sequence will be the same in every case. However, the primer pairs determined for the different random oligonucleotides will vary because the regions flanking the common probe region are different, being created randomly. The best optimal primer pair is chosen for use, even if this pair has a higher penalty score than the best non-optimal pair.

It will be apparent that the insertion of the probe sequence into a random oligonucleotide creates a sequence larger than 100 bases. This can be remedied in two ways. First, the random oligonucleotides generated in the first stage can be chosen to be about 70 bases in length, instead of 100 bases. Thus, insertion of the probe sequence will allow the entire length of the sequence to be less than about 100 bases in length, and the "Find Primers/Probe Now" routine can be applied to this sequence.. This however does not always give satisfactory results as the position at which the probe is inserted will affect the outcome of the primer finding routine. In some cases, no satisfactory primer pairs can be generated, and the particular random oligonucleotide will need to be discarded and the above procedure attempted on the next random oligonucleotide.

Alternatively, the random oligonucleotides generated in the first stage can be maintained at 100 bases in length. The probe sequence is inserted into the random oligonucleotide sequences, and once satisfactory primer pairs have been established, the resulting oligonucleotide can be edited in the "Primer Express" software to remove any redundant sequences. For example, it may be found that there are extraneous sequences present in the oligonucleotide lying outside the primer sequences. In other words, the primer sequences may not be at the extreme ends of the oligonucleotide sequence. In this case, the oligonucleotide may be edited to remove these sequences to reduce the length of the oligonucleotide, and the "Find Primers/Probe Now" routine can be undertaken again on the edited sequence.

It should be noted that the use of the "Primer Express" software in designing oligonucleotide primers and probes should only be viewed as an aid to maximise the chances of the 5' nuclease assay working well. The only absolute criterion to be applied is whether the assay actually works in reality. Thus, only a probe sequence that has performed well in an assay should be selected as the common probe sequence, and an untried probe sequence should never be used as the basis for tag design. Even if the probe works well with one particular set of primers against one specific target, it may not always work well. All DNA designs should be tried before the tag is released for commercial purposes.

The oligonucleotide tags, primers and probe sequences are then ordered commercially from custom synthesis facilities. In order to minimise the risk of contamination, different synthesis houses should be used for the synthesis of the oligonucleotide tags and primers. Otherwise primers can be contaminated with various tags, leading to "negative" controls giving a positive signal and a consequent decrease in the sensitivity of detection. Ideally, a third synthesis house should be used for the production of the probe. The labelled probes should be provided in buffer and should be protected from light.

The assay conditions should be standardised by using a single buffer mixture mix, with the only variants being the primers used and the sample of the material to be analysed. Since the 5' nuclease assay is based on polymerase chain reaction, the requirements for establishing a successful PCR assays should be adhered to. These requirements have been set out in detail in various publications, and it is considered that the person skilled in the art will be aware of these requirements.

The 5' nuclease assay is conducted using the same components as normal PCR except for the addition of the labelled probe. The PCR components are purchased from PE-Applied Biosystems as the "Taqman PCR Core Reagent Kit" which has part number N8080288. The components of the kit are as follows: Buffer A at 10X stock concentration for buffering pH and providing reference fluorescent dye, magnesium chloride at 25mM stock concentration for providing Mg²⁺ ions required for activity of DNA polymerase, dATP at 10mM stock concentration (adenine nucleotide required for DNA extension), dUTP at 10mM stock concentration (uracil nucleotide required for DNA extension), dGTP at 10mM stock concentration (guanine nucleotide required for DNA extension), dCTP at 10mM stock concentration (cytosine nucleotide required for DNA extension), and uracil N glycosidase (UNG) at 1 Unit/microlitre to prevent DNA carry-over from one assay to the next. The UNG enzyme is inactivated during the PCR reaction but will hydrolyse any DNA containing uracil at the start of the PCR process. Thus, the enzyme prevents any DNA from being carried over from one PCR reaction to the next. The PCR vials should not be opened to prevent DNA carry-over.

The oligonucleotide primers and the labelled probes should be formulated as 5 micromolar stock solutions in a suitable buffer which has the following composition: 1 mM Tris-HCl at pH 8.3, 5 mM KCl and 0.2mM magnesium chloride. The primers and probe solutions are aliquotted into a number of vials and stored frozen until required. No vial should be allowed to go through more than 5 freeze/thaw cycles.

The "Taqman PCR Core Reagent Kit" and the 5 micromolar probe stock solutions are then mixed together as follow: 100 microlitres of Buffer A, 220 microlitres of magnesium chloride, 20 microlitres of dATP, 20 microlitres of dUTP, 20 microlitres of dGTP, 20 microlitres of dCTP, 5 microlitres of AmpliTaq Gold *(Taq* DNA polymerase), 10 microlitres of UNG, 25 microlitres of labelled probe and 120 microlitres of water. The resulting 560 microlitres is sufficient for 40 assays if 14 microlitres is used per assay vial. To each of the assay vials is then added 4.5 microlitres of each of the forward and reverse primers, 5 microlitres of the 5 micromolar stock solutions described above and 2 microlitres of the sample containing the tag to be analysed. The resulting solution has the following final concentrations of each component: 1X Buffer A, 5 mM magnesium chloride, 0.2 mM dATP, 0.2 mM dUTP, 0.2 mM dGTP, 0.2 mM dCTP, 0.025 Units/microlitre AmpliTaq Gold, 0.05 Units/microlitre UNG, 0.125 micromolar labelled probe, 0.9 micromolar forward primer and 0.9 micromolar reverse primer. The final concentration of the target tag is unknown.

Either optical 96-well plates or optical tubes may be used as reaction containers. The former is catalogue number N8010560 and the latter catalogue number N8010933 available from PE-Applied Biosystems. Both of these require optical caps (catalogue number N8010935 from PE-Applied Biosystems). The ABI Prism 7700 Sequence Detector, made by PE-Applied Biosystems, is used for conducting the PCR and detecting fluorescent signal and should be operated in accordance with the manufacturer's instructions.

## Claims

1. A method of detecting whether a material has been marked with a nucleic acid tag, the method comprising the steps of:
(a) providing a pool of nucleic acid tags, each nucleic acid tag comprising a generic region having a sequence present in all nucleic acid tags within the pool, the generic region being flanked by code regions having identifying sequences for the tag;
(b) selecting a particular nucleic acid tag from within the pool;
(c) adding or applying a marker comprising the selected nucleic acid tag to the material; and
(d) contacting the nucleic acid tag with a labelled oligonucleotide probe capable of binding the generic region of the nucleic acid tag; and
(e) detecting hybridisation of the oligonucleotide probe to the generic region of the nucleic acid tag, thereby indicating that the material was marked with the nucleic acid tag.

2. A method as claimed in any preceding claim, in which the nucleic acid tag consists of DNA.

3. A method as claimed in any preceding claim, in which the nucleic acid tag is a single stranded DNA oligonucleotide.

## Patentansprüche

1. Verfahren zum Detektieren, ob ein Material mit einer Nukleinsäuremarkierung markiert worden ist, wobei das Verfahren die Schritte aufweist:
(a) Bereitstellen eines Pools von Nukleinsäuremarkierungen, wobei jede Nukleinsäuremarkierung eine generische Region mit einer Sequenz aufweist, die in allen Nukleinsäuremarkierungen in dem Pool vorhanden ist, wobei die generische Region durch Code-Regionen mit identifizierenden Sequenzen für die Markierung flankiert ist;
(b) Auswählen einer speziellen Nukleinsäuremarkierung aus dem Pool; und
(c) Hinzufügen oder Aufbringen eines Markers mit der ausgewählten Nukleinsäuremarkierung auf das Material;
(d) Inkontaktbringen der Nukleinsäuremarkierung mit einer gekennzeichneten Oligonukleotidsonde, der die generische Region der Nukleinsäuremarkierung binden kann; und
(e) Detektieren einer Hybridisierung der Oligonukleotidsonde an die generische Region der Nukleinsäuremarkierung, wodurch angegeben wird, dass das Material mit der Nukleinsäuremarkierung markiert wurde.

2. Verfahren nach Anspruch 1, in welchem die Nukleinsäuremarkierung aus DNA besteht.

3. Verfahren nach Anspruch 1 oder Anspruch 2, in welchem die Nukleinsäuremarkierung ein einzelsträngiges DNA-Oligonukleotid ist.

## Revendications

1. Procédé pour détecter si un matériau a été marqué avec une étiquette d'acide nucléique, le procédé comprenant les étapes consistant à :
(a) fournir un pool d'étiquettes d'acide nucléique, chaque étiquette d'acide nucléique comprenant une région générique ayant une séquence présente dans toutes les étiquettes d'acide nucléique à l'intérieur du pool, la région générique étant flanquée par des régions codantes ayant des séquences d'identification pour l'étiquette ;
(b) sélectionner une étiquette d'acide nucléique particulière dans le pool ; et
(c) ajouter ou appliquer un marqueur comprenant l'étiquette d'acide nucléique sélectionnée au matériau ;
(d) mettre en contact l'étiquette d'acide nucléique avec une sonde d'oligonucléotide marquée apte à se lier à la région générique de l'étiquette d'acide nucléique ; et
(e) détecter l'hybridation de la sonde d'oligonucléotide à la région générique de l'étiquette d'acide nucléique, indiquant ainsi que le matériau était marqué avec l'étiquette d'acide nucléique.

2. Procédé selon la revendication 1, dans lequel l'étiquette d'acide nucléique consiste en ADN.

3. Procédé selon la revendication 1 ou la revendication 2, dans lequel l'étiquette d'acide nucléique est un oligonucléotide ADN simple brin.
